(19) Europäisches Patentamt
European Patent Office
Office européen des brevets

(11) EP 4 362 993 B1

(12) # EUROPEAN PATENT SPECIFICATION

(45) Date of publication and mention of the grant of the patent:
**22.04.2026 Bulletin 2026/17**

(21) Application number: **22741238.4**

(22) Date of filing: **30.06.2022**

(51) International Patent Classification (IPC):
***A61L 27/20*** *(2006.01)* ***A61L 27/52*** *(2006.01)*
***A61K 8/73*** *(2006.01)* ***A61Q 19/08*** *(2006.01)*

(52) Cooperative Patent Classification (CPC):
(C-Sets available)
**A61L 27/52; A61K 8/735; A61L 27/20; A61Q 19/08;** A61K 2800/91; A61L 2300/402; A61L 2400/06; A61L 2430/34; A61L 2430/40
(Cont.)

(86) International application number:
**PCT/EP2022/068119**

(87) International publication number:
**WO 2023/275278 (05.01.2023 Gazette 2022/01)**

(54) **HYALURONIC ACID DERMAL FILLERS CROSSLINKED WITH POLYETHYLENE GLYCOL DIGLYCIDYL ETHER, METHOD FOR MAKING SAME AND USES THEREOF**

MIT POLYETHYLENGLYKOLDIGLYCIDYLETHER VERNETZTE, DERMALE HYALURONSÄUREFÜLLSTOFFE, VERFAHREN ZUR HERSTELLUNG DAVON UND VERWENDUNGEN DAVON

CHARGES DERMIQUES D'ACIDE HYALURONIQUE RÉTICULÉES AVEC DE L'ÉTHER DIGLYCIDYLIQUE DE POLYÉTHYLÈNE GLYCOL, LEUR PROCÉDÉ DE FABRICATION ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2021 EP 21182833**

(43) Date of publication of application:
**08.05.2024 Bulletin 2024/19**

(73) Proprietor: **Merz Pharma GmbH & Co. KGaA**
**60318 Frankfurt am Main (DE)**

(72) Inventors:
- **HÖNNSCHEIDT, Christoph**
  **60318 Frankfurt am Main (DE)**
- **EL-BANNA, Radia**
  **60318 Frankfurt am Main (DE)**

(74) Representative: **Fritsche, Erich Roland**
**Wallinger Ricker Schlotter Tostmann**
**Patent- und Rechtsanwälte Partnerschaft mbB**
**Zweibrückenstraße 5-7**
**80331 München (DE)**

(56) References cited:
**WO-A1-2018/083195** **WO-A1-2019/005848**
**WO-A1-2020/030629** **RU-C1- 2 582 702**

- **MONTICELLI DAMIANO ET AL: "Chemical Characterization of Hydrogels Crosslinked with Polyethylene Glycol for Soft Tissue Augmentation", vol. 7, no. 7, 14 April 2019 (2019-04-14), pages 1077 - 1081, XP055870337, Retrieved from the Internet <URL:https://spiroski.migration.publicknowledgeproject.org/index.php/mjms/article/download/oamjms.2019.279/3203> DOI: 10.3889/oamjms.2019.279**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **HO-YONG LEE ET AL: "Enhancement of bio-stability and mechanical properties of hyaluronic acid hydrogels by tannic acid treatment", CARBOHYDRATE POLYMERS, vol. 186, 1 April 2018 (2018-04-01), GB, pages 290 - 298, XP055755171, ISSN: 0144-8617, DOI: 10.1016/ j.carbpol.2018.01.056**
- **JEONG CHANG HEE ET AL: "In vitro toxicity assessment of crosslinking agents used in hyaluronic acid dermal filler", TOXICOLOGY IN VITRO, ELSEVIER SCIENCE, GB, vol. 70, 20 October 2020 (2020-10-20), XP086399805, ISSN: 0887-2333, [retrieved on 20201020], DOI: 10.1016/ J.TIV.2020.105034**



(52) Cooperative Patent Classification (CPC): (Cont.)

C-Sets
**A61L 27/20, C08L 5/08**

**Description**

## FIELD OF THE INVENTION

The present invention relates to a dermal filler composition based on crosslinked hyaluronic acid, wherein the hyaluronic acid is crosslinked with a high molecular weight polyethylene glycol diglycidyl ether (PEGDE) crosslinker having an average number of $-CH_2-CH_2-O-$ repetitive units of 10 to 50 and a low molecular weight PEGDE crosslinker having an average number of $-CH_2-CH_2-O-$ repetitive units of 1 to 7. Furthermore, the present invention relates to a method for preparing said dermal filler composition and its use for cosmetic purposes.

## BACKGROUND OF THE INVENTION

Hyaluronic acid (HA)-based hydrogels, obtained by crosslinking HA with 1,4-butanediol diglycidyl ether (BDDE), are the most used dermal fillers in aesthetic medicine procedures for facial rejuvenation. Hyaluronic acid (HA) is a polysaccharide composed of repeating units of N-acetyl-D-glucosamine and D-glucuronic acid and is naturally present in the human body. Its biocompatibility, hydrophilicity, non-immunogenic and unique viscoelasticity makes HA an ideal material for dermal fillers. However, non-crosslinked HA is rapidly degraded *in vivo* by hyaluronidase. HA is therefore commonly crosslinked using a crosslinking agent. The crosslinked HA product is characterized by an increased *in vivo* longevity and excellent mechanical and rheological properties for use as a dermal filler.

There are many different crosslinkers known in the art, but BDDE is by far the most commonly used crosslinker for dermal fillers. Under alkaline reaction conditions, the epoxide groups of BDDE preferentially react with the primary hydroxyl group of HA to form stable ether bonds. As a result, the HA chains are linked to form a stable three-dimensional network structure. However, BDDE has also some disadvantages. For example, any BDDE-crosslinked HA product always contains some amounts of unreacted (i.e., "free") BDDE as well as partially hydrolyzed BDDE (i.e., epoxydioles). These "epoxide impurities" contain reactive epoxide groups and are generally considered toxic. Therefore, a laborious purification procedure needs to be set up to remove these impurities to the greatest possible extent. Furthermore, BDDE is a small diepoxide molecule with no variability in its structure. Consequently, it is only possible to influence the properties of the final gel product (e.g., strength and cohesivity) via the amount of BDDE and the degree of crosslinking.

More recently, the use of PEGDE (polyethylene glycol diethyl ether) in the production of PEG-crosslinked HA-based fillers has been described (see WO 2019/130360). Another dermal filler formulation composed of hyaluronic acid crosslinked with PEGDE having an average molecular weight of 500 is described in WO 2019/005848. Moreover, PEG-crosslinked fillers are now also commercially available under the brand name NEAUVIA® (MatexLab SA, Switzerland). The NEAUVIA® fillers have been the subject of various studies reported in, e.g., Zerbinati et al., J. Biol. Regul. Homeost., Vol. 31, no. 2 (S2), 79-85 (2017), Zerbinati et al., J. Biol. Regul. Homeost., Vol. 31, no. 2 (S2), 87-90 (2017), Zerbinati et al., J. Biol. Regul. Homeost., Vol. 31, no. 2 (S2), 153-161 (2017), and Monticelli, Open Access Maced. J. Med. Sci., 7(7), 1077-1081 (2019)). According to these studies, the PEG-crosslinked (or "PEGylated") NEAUVIA® fillers integrate well into the connective tissue and provide a long-lasting filling effect.

Notwithstanding recent advances known to those skilled in the field of dermal fillers, there is an ongoing interest in filler designs to meet specific cosmetic needs. In other words, there is a continuing need for improvements to and/or alternative formulations for crosslinked HA-based dermal fillers that address the specific requirements necessary for a particular cosmetic use.

## OBJECT OF THE INVENTION

The object of the present invention is to provide a crosslinked HA-based dermal filler with a superior safety profile and well-balanced rheological properties.

## SUMMARY OF THE INVENTION

The above object is solved by using high molecular weight (HMW) polyethylene glycol diglycidyl ether (PEGDE) and low molecular weight (LMW) polyethylene glycol diglycidyl ether (PEGDE) as crosslinking agent in the preparation of HA-based dermal fillers. It was found that HMW-PEGDE enables the preparation of a HA-based dermal filler composition that has desirable rheological (e.g., complex viscosity $\eta^*$, elastic modulus G', viscous modulus G", loss modulus tan$\delta$, cohesivity, spreadability) and injectability properties (e.g., extrusion force). It was further found that the use of HMW-PEGDE results in crosslinked HA gels that are highly stable against thermal degradation, such as the thermal degradation encountered during autoclaving.

Advantageously, the HMW-PEGDE further allows for the reduction of the quantity of reactive epoxide groups necessary for crosslinking, which ultimately results in final crosslinked HA products with decreased levels of unwanted

toxic epoxide groups. In addition, unlike BDDE which is a single molecule, PEGDE can have different average molecular weights depending on the average number of repetitive $-CH_2-CH_2-O-$ units. Therefore, depending on the respective molecular weight of PEGDE, different properties of the final product are obtained. Hence, the use of HMW-PEGDEs with varying average molecular weights enables the preparation of HA-based dermal fillers with tailored properties tuned to meet specific clinical needs, e.g., to match aesthetic needs of patients with their expectations.

In a first aspect, the present invention provides a dermal filler composition based on crosslinked hyaluronic acid, wherein the hyaluronic acid is crosslinked with a high molecular weight PEGDE crosslinker of formula (I)

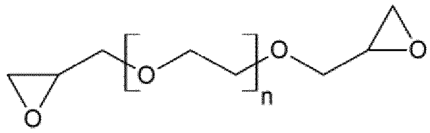

(I),

with an average number of repetitive units (n) of n = 10 to 50, more preferably 12 to 30, and a low molecular weight PEGDE crosslinker of formula (I) with an average number of repetitive units (n) of n = 1 to 7.

In a second aspect, the present invention provides a method for making a dermal filler composition according to the first aspect of the invention comprising the steps of:

(a) providing an aqueous mixture comprising hyaluronic acid (HA) in the non-crosslinked state and a polyethylene glycol diglycidyl ether (PEGDE) crosslinker,
(b) reacting the aqueous mixture to obtain a PEG-crosslinked HA gel product,
(c) purifying the PEG-crosslinked HA gel product, and
(d) sterilizing the PEG-crosslinked HA gel product to obtain the dermal filler composition.

The step of purifying the PEG-crosslinked HA gel product is preferably conducted by dynamic cross-flow filtration (DCF), and the step of sterilizing is preferably conducted by moist heat such as autoclaving. The purification step may be preceded by a neutralizing step, and optionally a swelling step.

In a third aspect, the present invention provides a dermal filler composition obtainable by the method according to the present invention.

In a fourth aspect, the present invention provides to a kit comprising a dermal filler composition according to the first and/or third aspect of the invention, and optionally instructions for use.

In a fifth aspect, the present invention relates to the use of a dermal filler composition according to the present invention (i.e., according to the first and/or third aspect of the invention) for cosmetic applications.

The cosmetic applications are not particularly limited and generally include dermal filler injections into the skin to restore, fill or augment the volume of skin tissue. Exemplary cosmetic applications include, but are not limited to, cosmetic treatments of facial wrinkles, wrinkles of other body parts such as hands, upper arms and décolettage, as well as skin rejuvenation, facial contouring, body contouring, and body filling.

In a sixth aspect, the present invention provides a method for replacing or filling of a biological tissue or increasing the volume of a biological tissue for cosmetic purposes, e.g., for cosmetic treatments as those mentioned above in connection with the fifth aspect, comprising administering to a subject in need thereof an effective amount of the dermal filler composition according to the present invention.

Preferred embodiments of the present invention are set forth in the appended claims. Further embodiments and other objects, advantages and features of the present invention will become apparent from the following detailed description of the invention, the accompanying figure, and the examples.

## BRIEF DESCRIPTION OF THE FIGURE

The present invention is further illustrated by reference to the following figure:

**FIG. 1** shows a G" versus log(G') plot (G' = elastic modulus; G" = viscous modulus) measured at 1 Hz for PEG(500)-crosslinked HA gels with 20 mg/ml or 26 g/ml HA (squares), PEG(1,000)-crosslinked HA gels with 20 mg/ml or 26 g/ml HA(dots), and PEG(500/1,000)-crosslinked HA gels with 20 mg/ml or 26 g/ml HA (triangles). The PEG(500/1,000)-crosslinked HA gel is made using a LMW-PEGDE with a molecular weight of 500 and a HMW-PEGDE with a molecular weight of 1,000 in a weight ratio of 3:1.

## DETAILED DESCRIPTION OF THE INVENTION

The present invention is based on the surprising finding that the use of high molecular weight (HMW)

polyethylene glycol diglycidyl ether (PEGDE) as an alternative to the commonly used BDDE crosslinker enables the manufacturing of a crosslinked HA gel that exhibits unique and suitable properties for use as a dermal filler, such as a sufficiently high modulus of elasticity (G') in combination with a low injection force through fine needles, and good flow characteristics. In addition to these favorable rheological and injectability properties, the HMW-PEGDE was found to provide crosslinked HA gels that are highly stable against thermal degradation, such as the thermal degradation encountered during autoclaving.

In particular, the use of HMW-PEGDE as crosslinker enables the production of "strong" gels (i.e., gels with high G' and comparably low G", thus lower tanδ) or "weak" or "soft" gels (i.e., gels with low G' and comparably high G", thus higher tanδ) with differing properties such as relatively low or relatively high cohesivity, respectively. For example, the use of HMW-PEGDE allows making a soft, highly cohesive gel, which also shows a high stability. In contrast, with BDDE-crosslinked HA gels, the gel strength (i.e., strong/weak) always determines the cohesivity. This is, a weak BDDE-crosslinked HA gel will also exhibit a relatively low cohesivity (and a reduced stability).

Moreover, the HMW-PEGDE allows for the reduction of reactive epoxide groups because a lower molar amount of HMW-PEGDE than BDDE is required to achieve the same rheological properties (e.g., G' and cohesivity). In other words, less reactive epoxide groups are involved in the crosslinking reaction, which translates into less toxic epoxide groups in the final purified HA gel product. This in turn allows for the administration of increased volumes of dermal filler as required in body filling or contouring applications.

Furthermore, unlike BDDE, HMW-PEGDE may have different molecular weights depending on the number of repetitive $-CH_2-CH_2-O-$ units, which impart different properties to the final product. Hence, the use of different HMW-PEGDE with varying molecular weights enables the preparation of HA-based dermal fillers with tailored properties tuned to match specific clinical needs.

In a first aspect, the present invention relates to a dermal filler composition comprising hyaluronic acid (HA) crosslinked with polyethylene glycol diglycidyl ether (PEGDE). The PEGDE crosslinker is a high molecular weight PEGDE (HMW-PEGDE) represented by formula (I) and a low molecular weight PEGDE (LMW-PEGDE) of formula (I),

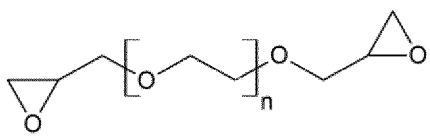

(I),

wherein n is the average number of repetitive units and, wherein the average number of repetitive units (n) of the HMW-PEGDE is between 10 and 50 and the average number of repetitive units (n) of the LMW-PEGDE is between 1 and 7.

It has been found that, in addition to a HMW-PEGDE crosslinker, one or more other PEGDE crosslinkers may be used to prepare the dermal filler composition of the present invention. Specifically, it has been found that the HA of the dermal filler composition may be crosslinked with a HMW-PEGDE crosslinker and at least one PEGDE crosslinker which differs from the HMW-PEGDE crosslinker in its average molecular weight and, thus, in its average number of repetitive units (n).

In the dermal filler composition of the present invention the hyaluronic acid is crosslinked with the HMW-PEGDE and, additionally, a low molecular weight PEGDE (LMW-PEGDE) crosslinker with an average number of repetitive units (n) of n = 1 to 7, preferably 2 to 7, more preferably 2 to 6.

Preferably, the average number of repetitive units (n) of the HMW-PEGDE is from 10, 11, 12, 13, 14, 15, 16, 17, 18, 19 or 20 to 50, or from 10, 11, 12, 13, 14, 15, 16, 17, 18, 19 or 20 to 45, or form 10, 11, 12, 13, 14, 15, 16, 17, 18, 19 or 20 to 40, or from 10, 11, 12, 13, 14, 15, 16, 17, 18, 19 or 20 to 35, or from 10, 11, 12, 13, 14, 15, 16, 17, 18, 19 or 20 to 30.

Preferably, the average number of repetitive units (n) of the LMW-PEGDE is from 2 to 7, 3 to 7, 4 to 7, 5 to 7, or 6 to 7, or from 1 to 6, 1 to 5, 1 to 4, 1 to 3, or 1 to 2, or from 2 to 6, or from 3 to 5.

Particularly suitable combinations of HMW-PEGDE and LMW-PEGDE include n = 10 to 30 / n = 1 to 7, n = 12 to 30 / n = 1 to 7, n = 12 to 25 / n = 2 to 7, n = 8 to 30 / n = 3 to 7, n = 10 to 25 / n = 4 to 7.

Within the framework of the present invention, the average molecular weight of PEGDE can be calculated from the average number of repetitive units (n) and vice versa using the following equation (1):

$$Mw\ (PEGDE) = 44.053 \times n + 130.1432 \qquad (1).$$

Thus, the average molecular weight (Mw) of PEGDE correlates with the average number of repetitive units (n) as follows: Mw = 200 corresponds to n = 1.586, Mw = 500 corresponds to n=8.396, Mw = 1000 corresponds to n=19.746, Mw = 2000 corresponds to n=42.446, Mw = 3000 corresponds to n=65.146, Mw = 4000 corresponds to n=87.845, and Mw =6000 corresponds to n=133.245.

The average number of repetitive units (n) can be experimentally determined by NMR spectroscopy as described

by Monticelli et al. (Open Access Maced. J. Med. Sci. 7(7):1077-1081 (2019)). PEGDE of varying molecular weights are commercially available. For example, a PEGDE(500) or PEG(500)DE product refers to PEGDE having an average molecular weight of 500 (average molecular mass of 500 g/mol). The average molecular weight of PEGDE referred to herein is generally the number average molecular weight (Mn), unless otherwise stated.

The number average molecular weight can be determined by various techniques (e.g., MALDI-MS, NMR, viscosity analysis). In the context of the present invention, the number average molecular weight (Mn) is generally determined by nuclear magnetic resonance (NMR) analysis, more specifically by NMR end-group analysis, an even more specifically by 1H NMR end-group analysis. As known to those skilled in the art, in 1H NMR spectroscopy, the area under each resonance signal is proportional to the molar concentration of the protons being analyzed. Number average molecular weight (Mn) determination by end-group analysis using 1H NMR therefore involves identifying and integrating distinguishable proton signals originating from end-groups and repeating units.

Within the framework of the present invention, if the used PEDGE is supplied by a commercial manufacturer, the molecular weight indicated by the manufacturer for a particular PEGDE product is deemed to be the average molecular weight referred to herein (in place of or alternative to the molecular weight definitions given herein above), which can be used to calculate the average number of repetitive units (n) by using the above equation (1).

The expression "is crosslinked with" or "crosslinked with", as used herein, is not intended to imply any restrictions or limitations regarding the crosslinking process and covers, in particular, any number and/or order of crosslinking steps, unless otherwise stated. This means that HA crosslinked in the presence of HMW-PEGDE and LMW-PEGDE as well as double-crosslinked or triple-crosslinked HA gels are within the meaning of said terms. For example, a dermal filler made by crosslinking a first portion of HA with a first crosslinker, adding a second portion of HA and crosslinking with the first crosslinker or a second crosslinker is encompassed. Also, crosslinking a first portion of HA with a first crosslinker and, separately therefrom, crosslinking a second portion of HA with the same first or a second crosslinker, followed by mixing the two crosslinked HA portions, and optionally co-crosslinking the mixture with the first or second crosslinker, is within the meaning of the present invention.

The use of PEGDE as a crosslinker results in covalent crosslinks between separate HA molecules (intermolecular crosslinks) and/or covalent crosslinks within the same HA molecule (intramolecular crosslinks). The crosslinked product is generally in the form of a three-dimensional network or "gel" structure. The term "gel", as used herein, usually refers to a material having fluidity at room or body temperature between that of a liquid and solid. Since the gel is present in water and contains absorbed water molecules, it is also referred to as "hydrogel" herein.

The term "dermal filler" or "dermal filler composition", as used herein, is intended to refer to a material designed to add volume to, or replace or augment volume of, soft tissue areas of skin. Generally, the dermal filler described herein is injectable, i.e., can be dispensed from syringes or similar devices under normal conditions under normal pressure to the desired target site (e.g., into the dermis and hypodermis). It is generally sterile and in the form of a gel, more specifically in the form of a hydrogel.

The dermal filler composition of present invention is generally cohesive. The term "cohesive" or "cohesivity" within the meaning of the present invention is defined as the capacity of a material (e.g., of a hydrogel) not to dissociate because of the affinity of its molecules for each other. This is, cohesivity is important with respect to gel integrity. Cohesivity is also important during the product distribution into the tissues of the treated area. Together with the viscosity level, the cohesivity profile defines the HA filler's capacity to remain at the injection site or to spread into the tissues. Cohesivity is difficult to accurately measure and there are various measurement protocols. For example, cohesivity may be determined using the Gavard-Sundaram Cohesivity Scale, a five-point visual reference scale (Sundaram et al., Plast. Reconstr. Surg. 136:678-686, 2015). Preferably, the dermal filler of the present invention has a cohesivity of 3, 4 or 5 on the five-point Gavard-Sundaram Cohesivity Scale.

The spreadability is another important parameter for characterizing a dermal filler and indicative of product distribution and tissue integration. Within the present invention, the spreadability parameter can be measured as explained in the examples by measuring the force required to perform compression (parameter "work" [mJ], i.e., the cumulative force that must be applied over the entire measurement). The spreadability, i.e., the measured force (mJ), indicates how strong the force must be in order to break the internal electrostatic bonds of the gel. The higher the measured force, the more stable are the internal forces in the gel. Strong internal forces are present in highly cohesive gels, which is why they form long filaments when pulled apart, as in the Gavard-Sundaram Cohesivity Scale. Thus, low spreadability is interrelated with high cohesivity and vice versa. Within the present invention, spreadability is not only a measure of product distribution and tissue integration but can also be taken as an indication of cohesivity.

The term "hyaluronic acid" or "HA", as used herein, includes hyaluronic acid, hyaluronate, and any of its hyaluronate salts, such as salts of hyaluronate and sodium, potassium, lithium, magnesium, calcium, or combinations thereof. The term "non-crosslinked", as used herein, refers to HA molecules that are not crosslinked, or very lightly crosslinked (very low degree of crosslinking or essentially uncrosslinked, e.g., a degree of modification of less than 1% or less than 0.1%). The molecular mass of HA is not particularly limited and may be, for example, between $2.0 \times 10^6$ Da and $5.0 \times 10^6$ Da, preferably between $5.0 \times 10^5$ Da and $4.0 \times 10^6$ Da, more preferably between $1.0 \times 10^6$ Da and $3.5 \times 10^6$ Da, and

most preferably between $2.0 \times 10^6$ Da and $3.0 \times 10^6$ Da.

It is further contemplated within the present invention that the HA of, or used for making, the dermal filler of the present invention is a mixture of two or more HA materials that differ from each other by their mean molecular weight. For example, the HA may comprise a mixture of high molecular weight HA having a molecular weight of, e.g., $2.0 \times 10^6$ Da to $4.0 \times 10^6$ Da, and a low molecular weight HA having a molecular weight of, e.g., $0.2 \times 10^6$ Da to $1.0 \times 10^6$ Da.

The ratio of the high molecular weight HA to the low molecular weight HA may be between 10:90 to 90:10, particularly from 50:50 to 90:10 or from 65:35 to 80:20. In one embodiment, 50% or more, 60% or more, 70% or more, 80% or more, 90% or more, or 100% of the HA is comprised of HA having a molecular weight greater than $1.0 \times 10^6$ Da, preferably at least $1.0 \times 10^6$ Da and more preferably $2.0 \times 10^6$ Da to $4.0 \times 10^6$ Da.

It is also within the scope of the present invention that the HA is not only mono-crosslinked, i.e., crosslinked in a single crosslinking reaction, but double-crosslinked or triple-crosslinked in similar terms to the procedures used for manufacturing the BDDE-crosslinked gels disclosed in, e.g., WO 2005/085329 (polydensified HA gels) and WO 2014/198406 A1 (triple-crosslinked HA gels). In a preferred embodiment, the HA is mono-crosslinked with the high molecular weight PEGDE and with the low molecular weight PEGDE.

Reference to "molecular weight" or "molecular mass" of HA are, for the purpose of the present invention, to be understood as indicating the viscosity average molecular mass ($M_v$). The viscosity average molecular mass can be calculated by relating the measured intrinsic viscosity ($\eta$) to the average molecular weight ($M_v$) by the following Mark-Houwink equation: $[\eta] = K \times M_v^a$, wherein [n] = intrinsic viscosity in $m^3/kg$, Mv = molecular mass, $K = 2.26 \times 10^{-5}\ m^3/kg$, and a = 0.796.

The intrinsic viscosity may be measured at 25°C using a buffer solution of 0.15 M sodium chloride in 0.01 M phosphate buffer solution (pH 7.0) by means of a suspended level viscometer (Ubbelohde type viscometer) according to the procedure defined in European Pharmacopoeia 7.0 (see sodium hyaluronate monograph 01/2011:1472)

In accordance with the present invention, the crosslinked hyaluronic acid is present in the dermal filler composition in an amount of between 1 mg/ml and 50 mg/ml, preferably between 5 mg/ml to 40 mg/ml, more preferably between 10 mg/ml and 35 mg/ml, still more preferably between 15 mg/ml and 30 mg/ml, and most preferably between 20 mg/ml and 25 mg/ml.

The degree of modification (MoD) of the crosslinked HA in the dermal filler composition, expressed as the ratio of the sum of mono- and double-crosslinked PEG crosslinkers to the sum of HA repeating disaccharide units, is preferably from 0.5% to 50%, from 1% to 20%, from 5% to 15%, or about 10%. The degree of modification can be determined by those skilled in the art using NMR in accordance with methods known in the art (see Monticelli et al., Open Access Macedonian J. Med. Sci. 7(7):1077-1081 (2019)). A skilled person will be able to identify the characteristic peaks of HA and of the PEG crosslinker to determine the degree of modification.

The dermal filler composition of the present invention exhibits a desired "smooth" appearance as desired and is generally cohesive. After heat sterilization (e.g., after autoclaving at 121°C for 10 min or at 127°C for 4 min), the dermal filler composition has one or more of the following properties: (a) a modulus of elasticity ($G'_{1Hz}$) of at least 50 Pa, particularly 50 Pa to 200 Pa or 100 Pa to 150 Pa, (b) a loss tangent ($\tan \delta_{1Hz}$) of less than 1.00, particularly less than 0.90, 0.80, 0.70, 0.60 or 0.50, and an injection force of less than 10 N, particularly less than 5 N, as determined through a 27G½ needle at an extrusion rate of about 0.21 mm/sec using a 1.0 ml glass syringe.

Within the context of the present invention, the dermal filler composition may comprise additional substances. In this respect, it is emphasized that the term "comprise", as used herein, for example in the context of "a compound comprising..." or "a method comprising...", is intended to encompass both the open-ended term "includes" and the closed-ended phrase "consisting of".

In particular, the dermal filler composition of the present invention may further comprise non-crosslinked HA, for example as a lubricant to improve the filler's rheological properties such as to lower its extrusion force. The molecular weight of the non-crosslinked HA is preferably between $3.0 \times 10^6$ Da and $4.0 \times 10^6$ Da, in particular between $1.0 \times 10^6$ Da and $3.0 \times 10^6$ Da.

The amount of non-crosslinked HA present in the dermal filler is not specifically limited but is typically less than 200 mg/g or less than 150 mg/g, more preferably less than 150 mg/g or less than 100 mg/g, and most preferably less than 50 mg/g or less than 20 mg/g, and typically at least 0.001 mg/g or at least 0.01 mg/g, preferably at least 0.1 mg/g or at least 1 mg/g, more preferably at least 2 mg/g or at least 5 mg/g, and most preferably at least 10 mg/g or at least 20 mg/g. Preferably, the amount of non-crosslinked HA present in the dermal filler is from 0.001 mg/g to 100 mg/g, in particular from 0.1 mg/g to 50 mg/g, and more particularly from 1 mg/g to 10 mg/g. Alternatively, the dermal filler composition of the present invention may also be free of any added non-crosslinked HA, i.e., being devoid or essentially devoid of any non-crosslinked HA.

The dermal filler of the present invention may also comprise at least one crosslinked and/or non-crosslinked polysaccharide other than HA. Non-limiting examples of such optional polysaccharides are cellulose and cellulose derivatives (e.g., carboxymethyl cellulose (CMC) or hydroxypropyl methylcellulose (HPMC) and glycosaminoglycans (GAG) such as heparosan, chondroitin sulfate, and dermatan sulfate). In one embodiment, the other polysaccharide is

(non-crosslinked) carboxymethyl cellulose (CMC). These other polysaccharides, in crosslinked or non-crosslinked form, may be present in the dermal filler in an amount of 20 wt.% or less, 15 wt.% or less, 10 wt.% or less, 8 wt.% or less, 6 wt.% or less, 4 wt.% or less, 2 wt.% or less, 1 wt.% or less or 0.01 wt.% or less. The lower limit is not specifically limited and may be, e.g., 0.001 wt.%. In a preferred embodiment, no polysaccharide(s) other than HA is present in the dermal filler composition of the present invention.

Furthermore, the dermal filler composition may optionally comprise an anesthetic, particularly a local anesthetic. The anesthetic is added for reducing pain caused by injection of the dermal filler. Generally, the total amount of anesthetic agent(s) included in the dermal filler composition of the present invention is in the range of 0.01 wt.% to 5 wt.% and, in particular, in the range of 0.1 wt.% to 2 wt.%.

Suitable local anesthetics for use herein include, but are not limited to, ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and salts thereof. Combinations of two or more of the mentioned anesthetic agents, for example a combination of lidocaine and other "caine"-anesthetic(s) like prilocaine, may also be used herein.

Preferably, the at least one anesthetic agent is lidocaine or a salt thereof, such as lidocaine hydrochloride (lidocaine HCl). The lidocaine concentration in the compositions described herein, in particular in the dermal filler composition, may be in the range of 0.05 wt.% to 5 wt.%, for example, from 0.1 wt.% to 2.0 wt.% or from 0.2% to 1.0 wt.%. Preferably, the lidocaine concentration is about 0.3 wt.%.

The dermal filler composition of the present invention may further comprise, but is not limited to, one or more compounds selected from the group consisting of antioxidants (e.g., ascorbic acid and derivatives thereof, tocopherols, carotenoids and derivatives thereof, retinol, glutathione, and ubiquinones), amino acids (e.g., glycine proline, lysine, arginine, leucine, isoleucine, and methionine), metal salts (e.g., a zinc salt), hydroxyapatite particles (e.g., calcium hydroxyapatite particles, preferably having a mean diameter of less than about 200 $\mu$m, e.g., 10 $\mu$m to 80 $\mu$m), as well as polyols and vitamins.

Suitable polyols include, but are not limited to, glycerol, mannitol, sorbitol, propylene glycol, erythritol, xylitol, maltitol, and lactitol. Particularly suitable for use herein is mannitol and glycerol. Further, the polyol is preferably glycol, optionally in combination with one or more of the aforementioned polyol compounds, in particular mannitol. The polyol(s) may, for example, be included in the injectable dermal filler composition in a concentration of 0.1% to 25% volume/volume or 1% to 20% volume/volume or 2% to 15% volume/volume, particularly in a concentration of 5% to 10% volume/volume.

Examples of vitamins include, but are not limited to, vitamin C, vitamin E and vitamins of the B group, i.e. one or more of $B_1$, $B_2$, $B_3$, $B_5$, $B_6$, $B_7$, $B_9$ and $B_{12}$ vitamins. The concentration of vitamin C or of vitamin E is preferably from about 0.01 mg/ml to about 10.0 mg/ml, more preferably from about 0.1 mg/ml to about 5.0 mg/ml, and the total concentration of the vitamins of the B group is preferably from about 0.01 mg/ml to about 10.0 mg/ml, more preferably from about 0.1 mg/ml to about 5.0 mg/ml. The total concentration of vitamins is generally 15 mg/ml or less, 10 mg/ml or less or 5 mg/ml or less. The vitamins may be present to stimulate and maintain cellular metabolism and, thus, to promote collagen production. Particularly preferred for use herein is vitamin C, vitamin E and vitamin $B_6$.

In a second aspect, the present invention relates to a method for making a dermal filler composition of the present invention comprising the steps of:

(a) providing an aqueous mixture comprising hyaluronic acid (HA) in the non-crosslinked state and a polyethylene glycol diglycidyl ether (PEGDE) crosslinker,
(b) reacting the aqueous mixture to obtain a PEG-crosslinked HA gel product,
(c) purifying the PEG-crosslinked HA gel product, and
(d) sterilizing the PEG-crosslinked HA gel product to obtain a dermal filler composition.

In step (a) the PEGDE may be provided, or added to the aqueous mixture, in any form. The aqueous mixture provided in step (a) and subjected to crosslinking may have a pH of 5 to 10, particularly 6 to 10, 7 to 10, or 7 to 9.

The aqueous mixture can be provided in any way and is not limited to a particular sequence of steps. For example, HA may be combined with an aqueous solution (e.g., water, phosphate buffer or phosphate buffered saline) to obtain an aqueous mixture of hydrated HA in the non-crosslinked state, followed by contacting the aqueous mixture with PEGDE. Alternatively, dry HA may be combined with an aqueous solution of PEGDE.

Step (b) comprises reacting the aqueous mixture with PEGDE under conditions that result in a PEG-crosslinked HA gel product. The term "PEG-crosslinked HA", as used herein, means HA crosslinked with PEGDE and may be used

synonymously to "PEGDE-crosslinked HA". The concentration of HA during crosslinking is typically between 50 mg/ml and 150 mg/ml, particularly between 75 mg/ml and 125 mg/ml, more particularly about 100 mg/ml.

The degree of crosslinking ($D_{cross}$), expressed as the molar ratio of PEGDE present in the aqueous mixture provided in step (a) and subjected to crosslinking in step (b) to repeating disaccharide units of HA present in the aqueous mixture provided in step (a) and subjected to crosslinking in step (b) is usually in the range of 0.1 to 20, more typically in the range of 1 to 15 or 2 to 10, in particular in the range of 3 to 5.

The crosslinking temperature and the crosslinking time are not particularly limited any may be selected by the skilled person depending on the respective application or use of the end product. The crosslinking temperature is generally at least 25°C, preferably at least 30°C or at least 35°C, more preferably at least 40°C, at least 45°C or at least 50°C. The upper limit of the crosslinking temperature is determined by the tendency of HA gels to degrade at excessively high temperatures. Those skilled in the art will be readily able to select appropriate temperatures to limit degradation to an acceptable level. Although not intended to be limiting, the upper temperature limit may be, for example, 65°C, 60°C, 55°C or 50°C. Thus, exemplary suitable temperature ranges are 25°C to 65°C, 30°C to 60°C, 35°C to 55°C, 40°C to 55°C, or 45°C to 50°C. The crosslinking time may be in the range of 0.25 to 10 hours, e.g., in the range of 1 to 5 hours.

After crosslinking, the reaction mixture obtained from step (b) is generally neutralized by adding an appropriate amount of an aqueous solution containing an acid or base. To this extent, a solution of NaOH or HCl in water, phosphate buffer or phosphate buffered saline may be used. However, the neutralizing step may also simultaneously occur with purification, such as in the course of cross-flow filtration or dialysis (see below).

Optionally, a swelling step may be carried out before purification (and after the neutralizing step, if present) in which the hydrated, crosslinked HA gels are allowed to swell in an aqueous solution (e.g. in a phosphate buffer, especially in a phosphate buffered saline) for a given time (e.g., 6 h to 50 h) and temperature (e.g., 2°C to 8°C). Alternatively, the swelling is not a separate step but may occur concurrently with the purification.

Step (c) of purifying the PEG-crosslinked HA gel product may be accomplished by methods such as dynamic cross-flow filtration (DCF) or dialysis. The purification step serves to remove undesired impurities, in particular unreacted PEGDE crosslinker, and/or to conduct a liquid exchange, e.g., a buffer (ex)change. Additional water or an aqueous buffer solution may be optionally added after purification (e.g., dialysis) to adjust the HA concentration of the gel as desired.

Preferably, the PEG-crosslinked HA gel is purified by dynamic cross-flow filtration (DCF). In this technology, the material to be filtered, i.e. the PEG-crosslinked HA gel, is fed into a DCF device at a feed overpressure. The material to be filtered may be fed to the DCF device from a feed reservoir, which is connected to an inlet. Inside the device, rotating membrane discs that typically overlap with each other generate a differential speed in relation to one another. Typically, ceramic filter membranes are employed, but other filter membranes such as metal filter membranes can also be used. The rotating discs creates a turbulent crossflow, whereby the formation of a clogging layer, which obstructs the permeate flow, can be avoided or significantly reduced. After feeding and concentrating the material to be filtered, i.e. the PEG-crosslinked HA gel, a diafiltration is usually carried out using, e.g., a phosphate buffer solution that is fed from a buffer reservoir to the inlet port and into the DCF device. The permeate is led to a permeate outlet, while the concentrated retentate is led to a retentate outlet and recovered. The diafiltration is carried out until residual crosslinkers and their degradation products or other impurities are below the desired limit.

The rotational speed of the membrane discs may be from 20/min to 500/min. Preferably, the rotational speed is in the range of 70/min to 300/min, more preferably from 100/min to 300/min. The overpressure of the material to be filtered inside the DCF device may be in the range of 0.5 to 6 bar. Preferably, the overpressure is in the range of 0.5 to 3 bar or 0.5 to 2 bar, more preferably in the range of 1 to 2 bar. The concentration of the crosslinked HA gel after DCF is typically in the range of 10 to 70 mg/g, preferably from 20 to 50 mg/g, more preferably from 20 to 35 mg/g.

Within the present invention, the use of DCF is advantageous because it provides the highest yields, even for very viscous material like concentrated crosslinked HA gel compositions. Further, the residence times are short, making the DCF process much faster than dialysis, and the purity of the concentrated end product is high. Suitably, the DCF process may be carried out, and using a DCF device, as described in WO 2020/030629.

Purification may, however, also be carried out using dialysis. The dialysis is usually carried out at low temperatures, e.g., at a temperature of about 2°C to 8°C, but higher temperatures may also be used. Generally, the dialysis is carried out for 6 h to 96 h, typically for about 12 h to 50 h, with multiple buffer changes, and the volume of the dialysis buffer is commonly at least 50 to 200 times the volume of the gel sample. Other suitable conditions for dialysis will be readily apparent to those skilled in the art. The dialysis buffer used for the dialysis may be, for example, a phosphate buffer such as a phosphate buffered saline, which preferably contains sodium chloride in an amount so that the osmolality of the dialysis buffer is between about 250 mOsm/kg to 350 mOsm/kg. The pH of the dialysis buffer may be from 6.0 to 8.0, preferably from 6.5 to 7.8, more preferably from 6.8 to 7.4.

It is also contemplated within the present invention that the method optionally comprises adding non-crosslinked HA and/or an anesthetic, particularly lidocaine, to the PEG-crosslinked HA gel product before sterilization. Furthermore, the pH may be adjusted to a desired value either before or after the addition of an anesthetic like lidocaine. Moreover, the method usually comprises one or several homogenization steps, e.g., mechanical stirring step(s), sieving step(s),

screening step(s) and the like, such as before or after dialysis and prior to sterilization.

In step (d), the PEG-crosslinked crosslinked HA gel is sterilized to obtain a sterile dermal filler composition. This step is usually carried out by subjecting the crosslinked HA gel to sterilization by moist heat. Sterilization may be, for example, accomplished by autoclaving the gel under appropriate conditions, such as at a temperature of 121°C to 130°C for 1 to 20 minutes, e.g. at 121°C for 5 minutes. Other suitable conditions are readily apparent to those skilled in the art.

Conveniently, the PEG-crosslinked HA gel may be sterilized in a syringe. Prior to filling the gel into the syringe, the gel is usually homogenized. The resulting sterile pre-filled syringes are convenient forms for intradermal administration by injection, especially in the ready-to-use version.

As explained above in connection with the dermal filler composition according to the first aspect of the invention, the dermal filler composition may optionally contain one or more additional polymers, compounds or additives, including non-crosslinked HA, crosslinked and/or non-crosslinked polysaccharides other than HA, local anesthetics such as lidocaine, antioxidants, amino acids, metal salts, hydroxyapatite particles, polyols, and vitamins.

These optional compounds may be added to the crosslinked HA gel at any appropriate process stage before final sterilization but are preferably added after dialysis and before sterilization. The explanations and definitions given above with respect to these additional polymers, compounds or additives apply, mutatis mutandis, to the method of the present invention.

In a third aspect, the present invention relates to a dermal filler composition obtainable by the method according to the present invention. In particular, this dermal filler composition is a dermal filler preparation that has been sterilized by moist heat, as described herein above.

The conditions of moist heat sterilization are preferably selected such that the resulting dermal filler is sterile, and its rheological properties are not unduly decreased due to the exposure to high temperatures. For example, the modulus of elasticity (G') after sterilization is preferably not decreased by more than 60%, 50% or 40%, and more preferably not decreased by more than 30% or 20%, compared to the G' value before sterilization.

In a fourth aspect, the present invention relates to a kit comprising a dermal filler composition according to the present invention, more specifically according to the first and/or third aspect of the invention, and optionally instructions for use.

The kit preferably comprises a syringe prefilled with the dermal filler composition of the present invention. The instructions for use preferably prescribe that the intended use of the kit is for cosmetic applications, in particular those described herein. The term "cosmetic" is interchangeably used herein with the term "aesthetic".

In a fifth aspect, the present invention relates to the use of a dermal filler composition according to the present invention for cosmetic applications, e.g., for improving the visual appearance, including body or face contouring and body filing uses.

The cosmetic applications are not particularly limited and generally include dermal filler injections into the skin to restore, fill or augment the volume of skin tissue. Exemplary cosmetic applications include, but are not limited to, cosmetic treatments of facial lines and facial wrinkles (e.g., glabellar lines, nasolabial folds, chin folds, marionette lines, buccal commissures, peri-oral wrinkles, peri-lip wrinkles, crow's feet). Other exemplary cosmetic applications include treating the infraorbital region, wrinkles in the décolettage, neck wrinkles, wrinkles of the hands, wrinkles of the upper arms and/or improve skin hydration and skin texture.

In particular, the dermal filler composition according to the present invention may be used in the treatment of "superficial indications", including skin rejuvenation (e.g., use as a "skin booster") and body rejuvenation (superficial treatment of wrinkles at non-face body sites such as arms, hands and neck), and smoothing superficial wrinkles. Furthermore, the dermal filler composition according to the present invention may be used for facial contouring, i.e., to enhance cheekbones, define the jawline, lift the face, reshape the nose, plumb or reshape the lips, reshape the chin, and improve the face's symmetry. Furthermore, another indication of interest is body contouring or body filling for volume restoration or augmentation of various body parts, including breast and buttock augmentation.

In a sixth aspect, the present invention relates to a method for replacing or filling of a biological tissue or increasing the volume of a biological tissue for cosmetic purposes, in particular for cosmetic treatments of skin lines and wrinkles, comprising administering to a subject in need thereof an effective amount of the dermal filler composition according to the present invention.

The dermal filler composition of the present invention is generally administered by injection, more specifically by subcutaneous or intradermal injection, using techniques known in the art such as the serial puncture technique. In particular, the dermal filler composition may be injected into the dermis and/or the subcutis, preferably into the deep dermis and/or upper subcutis.

The term "effective amount", as used herein, is generally intended to refer to the amount of the dermal filler composition sufficient to effect beneficial or desired cosmetic (aesthetic) results. A "subject" in the sense of the present invention is any individual or patient, usually a human, in need of a treatment of a particular condition.

The present invention will now be further illustrated by the following, non-limiting example.

## EXAMPLES

The examples below demonstrate that the use of high-molecular weight PEGDE for crosslinking hyaluronic acid (HA) leads to beneficial rheological properties, e.g., high volumizing ability, cohesivity and stability, of the resulting crosslinked HA gel product.

*Rheological measurements*

Rheological measurements were carried out using an Anton Paar MCR 302 rheometer equipped with a cone of 1°, 50 mm diameter. The measurements were performed at a shear deformation of 0.1% and a frequency from 0.1 to 10 Hz with a constant gap of 0.1 mm at 25°C. From the frequency scan the elastic modulus (G'), the viscous modulus (G"), the loss tangent (tan delta, tan $\delta$ = G"/G'), and the complex viscosity ($\eta$) were determined.

*Measurement of extrusion force*

The extrusion force was measured using a Texture Analyser (TA-XT plus, Stable Micro Systems, Haslemere, Surrey, UK) through a TSK needle of 27G½ and 30G½, respectively, at a rate of 12.5 mm/min using a 1 ml siliconized COP syringe (syringe made of cycloolefin polymer) with an inner diameter of 5 mm, and sterilized by autoclaving (127°C, 4 minutes).

Measurement of spreadability

The spreadability test is based on the compression of a sample to a certain gap size with a constant velocity using a plate-plate system equipped rheometer. An Anton Paar MCR 302 rheometer with a PP35 measuring rheometer rotor (Anton Paar) and a I-PP80/PE-D80mmPEEK measuring rheometer plate (Anton Paar) was used. The gap size was 1 mm and the velocity (V) was 100 $\mu$m/s. 50 data points were recorded (0.06 s). All measurements were carried out at a temperature of 25°C.

For the measurement, the gel was extruded from its original container (a syringe) on the rheometer plate, and the normal force against the measuring gap was recorded. Two parameters were obtained from each measurement. The parameter "work" [mJ] is defined as energy required to perform compression and is calculated by integrating the obtained curve. The second parameter is the "Maximum Normal Force" (Max. $F_N$ [N]) which is the highest (last) point of normal force read at the end of the compression. All measurements were carried out in triplicate.

Example 1 (reference example)

*Preparation of PEGDE crosslinked HA gels*

Sodium hyaluronic acid (NaHA) with a molecular mass of 2.9 MDa and 2.4 MDa respectively, was pre-swollen in phosphate buffer (HA concentration of 140 mg/g) at 50°C for 40 minutes and then cooled down to below 5°C with mixing. A NaOH solution was added to obtain a gel cake with 1 % w/w% NaOH content and a HA concentration of 130 mg/g, followed by the addition of PEG-DE (PEG (500)-DE (M=500 g/mol), PEG (1,000)-DE (M=1,000 g/mol) or PEG (6,000)-DE (M=6,000 g/mol), all available from Sigma-Aldrich) in phosphate buffer to result in a HA cake of 100 mg HA/g and 0.5 to 4 w/w% PEGDE.

After mixing for 15 minutes the cake was covered with the extruder stamp to remove air, heated up to 40°C and allowed to crosslink for 3 hours at 40°C. Then, the crosslinked product was cooled down to below 5°C and the extruder stamp was removed. After mixing for 2 minutes a HCl/phosphate buffer was added for neutralization (pH = 7.0 $\pm$ 0.1) and mixed for 30 minutes. Thereafter, the gel was homogenized by sieving through a 32x32 mesh filter to obtain a homogeneous PEG-crosslinked HA cake.

The obtained PEG-crosslinked HA cake was then purified by dynamic cross-flow filtration (DCF) (ANDRITZ KMPT GmbH, Vierkirchen, System: type 152/014, 355 g chamber volume, 2 nm pore sized discs). To this end, 200 g of PEG-crosslinked HA cake was added into the chamber. The chamber was then filled with phosphate buffer, the temperature was set to 20 °C and the disc was rotated. Phosphate buffer was then continuously filled into the chamber at 1.5 bar pressure, and at least 10 chamber volumes (3.55 kg) filtrate was collected.

Finally, the purified PEG-crosslinked HA gel was removed from the chamber. The purified PEG-crosslinked HA gel was mixed with phosphate buffer and optionally with a lubrication phase (uncrosslinked HA). Then, the gel was shaken over night at room temperature. The HA concentration of the final gel was 20 mg/ml.

The PEG-crosslinked HA gel was then filled into a 1 ml syringe and sterilized by autoclaving (127°C, 4 minutes).

Example 2 (reference example)

*Rheological properties of PEGDE-crosslinked HA gels before and after sterilization*

The complex viscosity (η), elastic modulus (G') and viscous modulus (G") were measured before and after sterilization (127°C, 4 min). The loss tangent (tanδ) was calculated from the measured G' and G" as follows: tanδ = G"/G'. Additionally, the extrusion force (EF) through a 27G½ needle and a 30G½ needle, respectively, was measured after sterilization of the gels.

Furthermore, the spreadability (SP) of the gels after sterilization was measured by determining the "work" [mJ], i.e., the cumulative force that must be applied over the entire measurement. This is, the higher the measured "work", the lower the spreadability. The spreadability is an indication of cohesiveness, as explained herein above. Low spreadability (i.e. high "work" values [mJ] correlate with high cohesivity). The results are shown in TABLE 1.

TABLE 1. Rheological characteristics

| Gel | PEG Mw | [PEG] wt.-% | [HA]f mg/ml[1] | η* [Pa·s] | G' [Pa] | G" [Pa] | tanδ | $EF_{27G½}$ [N] | $EF_{30G½}$ [N] | SP[2] [mJ] |
|---|---|---|---|---|---|---|---|---|---|---|
| | | | | (before/after sterilization) | | | | (after sterilization) | | |
| 1 | 500 | 2.25 | 20.3 | 43<br>33 | 255<br>197 | 75<br>61 | 0.29<br>0.31 | 5.9 | 10.0 | 0.87 |
| 2 | | | 26.0 | 68<br>54 | 415<br>329 | 111<br>95 | 0.27<br>0.29 | 9.6 | 16.3 | 1.49 |
| 3 | | 4.00 | 18.7 | 85<br>66 | 523<br>405 | 114/8<br>5 | 0.22<br>0.21 | 6.7 | 11.5 | 1.00 |
| 4 | | | 25.8 | 117<br>90 | 729<br>555 | 107<br>88 | 0.15<br>0.16 | 11.6 | 21 | 1.65 |
| 5 | 1000 | 2.25 | 20.0 | 169<br>84 | 157<br>95 | 70<br>51 | 0.45<br>0.53 | 8.9 | 14.4 | 1.12 |
| 6 | | | 24.1 | 176<br>159 | 184<br>161 | 82<br>83 | 0.45<br>0.52 | 13.0 | 26.4 | 1.52 |
| 7 | | 4.00 | 19.2 | 34<br>28 | 207<br>170 | 63<br>51 | 0.31<br>0.30 | 7.0 | 13.0 | 0.97 |
| 8 | | | 23.7 | 61<br>39 | 374<br>234 | 95<br>72 | 0.25<br>0.31 | 10.9 | 19.4 | 1.42 |
| 9 | 6000 | 2.25 | 19.3 | 5.5<br>3.6 | 17<br>9 | 30<br>21 | 1.82<br>2.37 | 3.3 | 5.4 | 0.11 |
| 10 | | | 24.8 | 13<br>9.2 | 47<br>28 | 67<br>50 | 1.45<br>1.81 | 4.6 | 8.0 | 0.27 |
| 11 | | 4.00 | 15.4 | 3.2<br>1.4 | 9<br>2 | 18<br>8 | 2.09<br>3.7 | 2.7 | 4.6 | 0.05 |
| 12 | | | 21.2 | 8.2<br>3.5 | 27<br>7 | 44<br>21 | 1.6<br>2.9 | 3.8 | 5.9 | 0.10 |

[1] HA with a molecular weight of 2.9 MDa was used for all gels, except for gels 5 and 6 which were manufactured using HA with a molecular weight of 2.4 MDa.
[2] Shown are the measured "work" [mJ] values, i.e., the cumulative force that must be applied over the entire measurement. Thus, higher "work" [mJ] values indicate lower spreadability.

The above results show that crosslinking of HA with HMW-PEGDE leads to gels with good rheological properties (e.g., viscosity and lifting capacity) and high syringeability (i.e., low extrusion force). In addition, since the amount of reactive epoxide groups during crosslinking is reduced compared to the use of BDDE, higher filler volumes can be used which offers the possibility for use as body filler. Furthermore, the level of spreadability is in the desired range, which allows for a homogeneous distribution of the dermal filler in the tissue after injection, as intended for the respective application.

In addition, as can be seen from the values before and after sterilization, the PEG-crosslinked gels show high resistance to thermal degradation and, thus, are expected to have a long persistence in the skin tissue. This in turn results in longer patient satisfaction, in particular for superficial indications.

Moreover, in line with the above results and further confirmed by additional studies (results not shown), it was found that the molecular weight of the used PEGDE crosslinker and the molecular weight of the used HA are the two most important determinants of G', G", extrusion force and spreadability. More specifically, the following relationships were found (after sterilization): G' increase: low Mw(PEGDE) > high HA end concentration; G" increase: high HA end concentration > low Mw(PEGDE); extrusion force (EF) decrease: low HA end concentration > high Mw(PEGDE); spreadability (SP) increase: high Mw(PEGDE) > low HA end concentration. Thus, the use of polymeric PEGDE crosslinkers with different molecular weights generate multiplied options for end product characteristics.

Example 3 (reference example)

*Stability of PEGDE crosslinked HA*

A PEG(500)DE-crosslinked HA (Mw(HA): 2.4 MDa; PEG content: 2.4 wt.%; HA concentration in crosslinking mixture: 100.0 mg/ml) and a PEG(1000)DE-crosslinked HA (Mw(HA): 2.4 MDa; PEG content: 2.25 wt.%; HA concentration in crosslinking mixture: 100.0 mg/ml) were prepared analogously to Example 1 and subjected to an accelerated stability study at 40°C. The elastic modulus (G') was measured directly after sterilization (127°C, 4 min; $F0_{(avg)}$ = 25.5 min) and after storage at 40°C for 12 weeks. The results are shown in TABLE 2.

TABLE 2. Stability study

| Gel | Final [HA] (mg/g) | T0 G' (1Hz) [Pa] | 12 weeks, 40°C G' (1Hz) [Pa] | G' drop (%) |
|---|---|---|---|---|
| PEG(500)crosslinked HA | 20.0 | 183 | 164 | 10.9 |
| PEG(1000)crosslinked HA | 24.9 | 77 | 48 | 18.4 |

As can be seen from TABLE 2, PEG-crosslinked HA shows a high thermal stability (low G' drop). On the other hand, like BDDE-crosslinked HA, the PEG-crosslinked HA can still be degraded by the enzyme hyaluronidase (results not shown), avoiding a non-permanent behavior.

Example 4

*Mixing of high and low molecular weight PEGDEs*

A PEG-crosslinked HA gel was prepared as described in Example 1, except that two different PEGDE crosslinkers were used, i.e., PEG(500)DE (a low molecular weight PEGDE or "LMW-PEGDE") and PEG(1,000)DE (a high molecular weight PEGDE or "HMW-PEGDE"), in a weight ratio of 3:1. This gel is referred to as PEG(500/1,0000)DE-crosslinked HA gel). The overall PEG content (i.e. of both PEG(500)DE and PEG(1,000)DE) in the crosslinking mixture used for preparing this gel was 2.25 wt.%. The molecular weight of the used HA was 2.9 MDa, and the HA end concentration was 20 or 26 mg/ml.

In addition, a PEG(500)DE-crosslinked HA gel and a PEG(1,000) crosslinked gel were prepared in accordance with Example 1, with a PEG content of 2.25 wt.%, a molecular weight of the HA of 2.9 MDa), and a HA end concentration of 20 or 26 mg/ml.

The elastic modulus (G') of the resulting PEG-crosslinked HA gels was measured directly after sterilization (127°C, 4 min; $F0_{(avg)}$ = 25.5 min). The results are shown in **FIG. 1.**

As can be seen in FIG. 1, the data points obtained for the PEG(500/1,0000)DE-crosslinked HA gel (see, triangles) are located below the dashed line, which fits the data measured for the PEG(500)DE- and PEG(1,000)DE-crosslinked HA gels. In other words, G" surprisingly shows a non-linear behavior. The use of a HMW-PEGDE in combination with a LWM-PEGDE therefore offers further possibilities to provide HA fillers with novel properties for various cosmetic applications.

## Claims

1. A dermal filler composition based on crosslinked hyaluronic acid, wherein the hyaluronic acid is crosslinked with a high molecular weight PEGDE crosslinker of formula (I) with an average number of repetitive units (n) of n = 10 to 50 and a low molecular weight PEGDE crosslinker of formula (I) with an average number of repetitive units (n) of n = 1 to 7

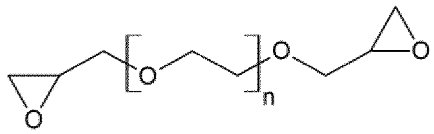

(I).

2. The dermal filler composition of claim 1, wherein n in formula (I) for the low molecular weight PEGDE crosslinker is 2 to 6.

3. The dermal filler composition of claim 1 or 2, wherein the degree of crosslinking, expressed as the molar ratio of PEGDE crosslinker subjected to crosslinking to repeating disaccharide units of HA subjected to crosslinking is from 0.1 to 20.

4. The dermal filler composition of any one of claims 1 to 3, wherein the dermal filler composition is made using a weight ratio of the first high molecular weight PEGDE crosslinker to the second low molecular weight PEGDE crosslinker of 95:5 to 5:95.

5. The dermal filler composition of any one of claims 1 to 4, wherein the hyaluronic acid comprises or consists of hyaluronic acid having an average molecular weight greater than 1.0 x $10^6$ Da or is a mixture of high molecular weight hyaluronic acid having an average molecular weight greater than 1.0 x $10^6$ Da and a low molecular weight hyaluronic acid having an average molecular weight of less than 1.0 x $10^6$ Da.

6. The dermal filler composition of any one of claims 1 to 5, wherein the hyaluronic acid is present in the dermal filler composition in an amount of between 10 mg/ml and 35 mg/ml.

7. The dermal filler composition of any one of claims 1 to 6, further comprising non-crosslinked HA or a local anesthetic agent, preferably lidocaine, or both non-crosslinked HA and a local anesthetic agent, preferably lidocaine.

8. A method for making a dermal filler composition of any one of claims 1 to 7 comprising the steps of:

(a) providing an aqueous mixture comprising hyaluronic acid (HA) in the non-crosslinked state and a polyethylene glycol diglycidyl ether (PEGDE crosslinker,
(b) reacting the aqueous mixture to obtain a PEG-crosslinked HA gel product,
(c) purifying the PEG-crosslinked HA gel product, and
(d) sterilizing the PEG-crosslinked HA gel product to obtain a dermal filler composition.

9. The method of claim 8, wherein the purification step (c) is carried out using dynamic cross-flow filtration (DCF).

10. A dermal filler composition obtainable by the method according to claim 8 or 9.

11. A kit comprising a dermal filler composition according to any one of claims 1 to 7 and 10, and optionally instructions for use.

12. Use of a dermal filler composition according to any one of claims 1 to 7 and 10 for cosmetic applications.

13. A method for replacing or filling of a biological tissue or increasing the volume of a biological tissue for cosmetic purposes, comprising administering to a subject in need thereof an effective amount of the dermal filler composition according to any one of claims 1 to 7 and 10.

**Patentansprüche**

1. Dermalfüllerzusammensetzung auf der Basis von vernetzter Hyaluronsäure, wobei die Hyaluronsäure mit einem hochmolekularen PEGDE-Vernetzer der Formel (I) mit einer mittleren Anzahl von Wiederholungseinheiten (n) von n = 10 bis 50 und einem niedermolekularen PEGDE-Vernetzer der Formel (I) mit einer mittleren Anzahl von Wiederholungseinheiten (n) von n = 1 bis 7 vernetzt ist

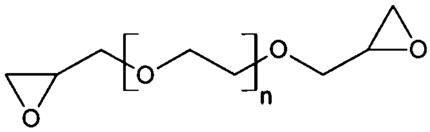

(I).

2. Dermalfüllerzusammensetzung nach Anspruch 1, wobei n in Formel (I) für den niedermolekularen PEGDE-Vernetzer 2 bis 6 beträgt.

3. Dermalfüllerzusammensetzung nach Anspruch 1 oder 2, wobei der Vernetzungsgrad, ausgedrückt als das molare Verhältnis von zu vernetzendem PEGDE-Vernetzer zu sich wiederholenden Disaccharideinheiten von zu vernetzendem HA, 0,1 bis 20 beträgt.

4. Dermalfüllerzusammensetzung nach einem der Ansprüche 1 bis 3, wobei die Dermalfüllerzusammensetzung unter Verwendung eines Gewichtsverhältnisses des ersten hochmolekularen PEGDE-Vernetzers zu dem zweiten niedermolekularen PEGDE-Vernetzer von 95:5 bis 5:95 hergestellt wird.

5. Dermalfüllerzusammensetzung nach einem der Ansprüche 1 bis 4, wobei die Hyaluronsäure Hyaluronsäure mit einem mittleren Molekulargewicht von mehr als 1,0 x $10^6$ Da umfasst oder daraus besteht, oder eine Mischung aus hochmolekularer Hyaluronsäure mit einem mittleren Molekulargewicht von mehr als 1,0 x $10^6$ Da und einer niedermolekularen Hyaluronsäure mit einem mittleren Molekulargewicht von weniger als 1,0 x $10^6$ Da ist.

6. Dermalfüllerzusammensetzung nach einem der Ansprüche 1 bis 5, wobei die Hyaluronsäure in der Dermalfüllerzusammensetzung in einer Menge zwischen 10 mg/ml und 35 mg/ml vorhanden ist.

7. Dermalfüllerzusammensetzung nach einem der Ansprüche 1 bis 6, die ferner nicht vernetzte HA oder ein Lokalanästhetikum, vorzugsweise Lidocain, oder sowohl nicht vernetzte HA als auch ein Lokalanästhetikum, vorzugsweise Lidocain, umfasst.

8. Verfahren zur Herstellung einer Dermalfüllerzusammensetzung nach einem der Ansprüche 1 bis 7, umfassend die Schritte:

(a) Bereitstellen einer wässrigen Mischung umfassend Hyaluronsäure (HA) im nicht vernetzten Zustand und einen Polyethylenglykoldiglycidylether (PEGDE) als Vernetzer,
(b) Umsetzen der wässrigen Mischung, um ein PEG-vernetztes HA-Gelprodukt zu erhalten,
(c) Aufreinigen des PEG-vernetzten HA-Gelprodukts, und
(d) Sterilisieren des PEG-vernetzten HA-Gelprodukts, um eine Dermalfüllerzusammensetzung zu erhalten.

9. Verfahren nach Anspruch 8, wobei der Aufreinigungsschritt (c) unter Verwendung der dynamischen Querstromfiltration (DCF) durchgeführt wird.

10. Dermalfüllerzusammensetzung erhältlich durch das Verfahren nach Anspruch 8 oder 9.

11. Kit umfassend eine Dermalfüllerzusammensetzung nach einem der Ansprüche 1 bis 7 und 10 und gegebenenfalls eine Gebrauchsanweisung.

12. Verwendung einer Dermalfüllerzusammensetzung nach einem der Ansprüche 1 bis 7 und 10 für kosmetische Anwendungen.

13. Verfahren zum Ersetzen oder Auffüllen eines biologischen Gewebes oder zur Vergrößerung des Volumens eines biologischen Gewebes zu kosmetischen Zwecken, umfassend die Verabreichung einer wirksamen Menge der Dermalfüllerzusammensetzung nach einem der Ansprüche 1 bis 7 und 10 an ein Subjekt, das dies benötigt.

## Revendications

1. Composition de produit de comblement dermique à base d’acide hyaluronique réticulé, dans laquelle l’acide hyaluronique est réticulé avec un agent de réticulation de PEGDE de haut poids moléculaire de formule (I) avec

un nombre moyen d'unités de répétition (n) n = 10 à 50 et un agent de réticulation de PEGDE de bas poids moléculaire de formule (I) avec un nombre moyen d'unités de répétition (n) n = 1 à 7

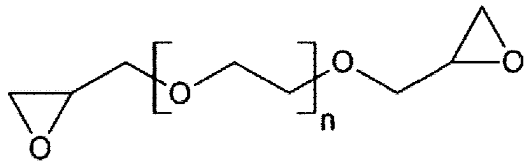

(I).

2. Composition de produit de comblement dermique selon la revendication 1, dans laquelle n dans la formule (I) pour l'agent de réticulation de PEGDE de bas poids moléculaire est compris entre 2 et 6.

3. Composition de produit de comblement dermique selon la revendication 1 ou 2, dans laquelle le taux de réticulation, exprimé comme le rapport molaire entre l'agent de réticulation de PEGDE soumis à réticulation et les unités disaccharidiques de répétition de l'acide hyaluronique HA soumis à réticulation, est compris entre 0,1 et 20.

4. Composition de produit de comblement dermique selon l'une quelconque des revendications 1 à 3, dans laquelle la composition de produit de comblement dermique est fabriquée en utilisant un rapport en poids entre le premier agent de réticulation de PEGDE de haut poids moléculaire et le deuxième agent de réticulation de PEGDE de bas poids moléculaire compris entre 95 : 5 et 5 : 95.

5. Composition de produit de comblement dermique selon l'une quelconque des revendications 1 à 4, dans laquelle l'acide hyaluronique comprend ou est constitué d'acide hyaluronique ayant un poids moléculaire moyen supérieur à $1{,}0 \times 10^6$ Da ou est un mélange d'acide hyaluronique de haut poids moléculaire ayant un poids moléculaire moyen supérieur à $1{,}0 \times 10^6$ Da et d'acide hyaluronique de bas poids moléculaire ayant un poids moléculaire moyen inférieur à $1{,}0 \times 10^6$ Da.

6. Composition de produit de comblement dermique selon l'une quelconque des revendications 1 à 5, dans laquelle l'acide hyaluronique est présent dans la composition de produit de comblement dermique en une quantité comprise entre 10 mg/ml et 35 mg/ml.

7. Composition de produit de comblement dermique selon l'une quelconque des revendications 1 à 6, comprenant en outre de l'acide hyaluronique HA non réticulé ou un agent anesthésique local, préférentiellement de la lidocaïne, ou à la fois de l'acide hyaluronique HA non réticulé et un agent anesthésique local, préférentiellement de la lidocaïne.

8. Procédé de fabrication d'une composition de produit de comblement dermique selon l'une quelconque des revendications 1 à 7 comprenant les étapes suivantes :

(a) disposer d'un mélange aqueux comprenant de l'acide hyaluronique (HA) sous la forme non réticulée et un agent de réticulation de polyéthylène glycol diglycidyl éther (PEGDE),
(b) faire réagir le mélange aqueux afin d'obtenir un produit de gel d'acide hyaluronique HA réticulé au PEG.
(c) purifier le produit de gel d'acide hyaluronique HA réticulé au PEG, et
(d) stériliser le produit de gel d'acide hyaluronique HA réticulé au PEG afin d'obtenir une composition de produit de comblement dermique.

9. Procédé selon la revendication 8, dans lequel l'étape de purification (c) est mise en œuvre à l'aide d'une filtration dynamique tangentielle (DCF).

10. Composition de produit de comblement dermique apte à être obtenue par le procédé selon la revendication 8 ou 9.

11. Kit comprenant une composition de produit de comblement dermique selon l'une quelconque des revendications 1 à 7 et 10 et éventuellement des instructions d'utilisation.

12. Utilisation d'une composition de produit de comblement dermique selon l'une quelconque des revendications 1 à 7 et 10 pour des applications cosmétiques.

13. Procédé pour le remplacement ou le comblement d'un tissu biologique ou l'augmentation du volume d'un tissu

biologique à des fins cosmétiques, comprenant l’administration à un sujet le nécessitant d’une quantité efficace de la composition de produit de comblement dermique selon l’une quelconque des revendications 1 à 7 et 10.

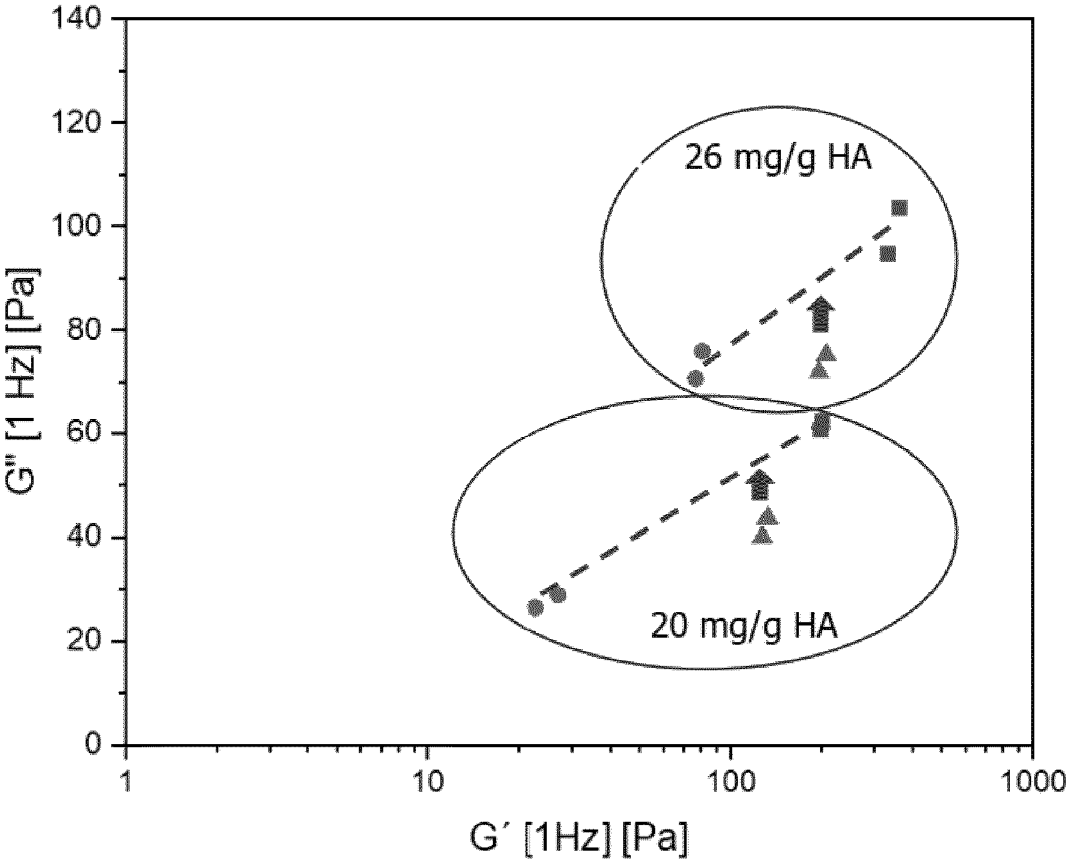
140
120
100
80
60
40
20
0
1
10
100
1000
G'' [1 Hz] [Pa]
G´ [1Hz] [Pa]
26 mg/g HA
20 mg/g HA

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019130360 A
- WO 2019005848 A
- WO 2005085329 A
- WO 2014198406 A1
- WO 2020030629 A


### Non-patent literature cited in the description


- **ZERBINATI et al.** *J. Biol. Regul. Homeost.*, 2017, vol. 31 (2), 79-85
- **ZERBINATI et al.** *J. Biol. Regul. Homeost.*, 2017, vol. 31 (2), 87-90
- **ZERBINATI et al.** *J. Biol. Regul. Homeost.*, 2017, vol. 31 (2), 153-161
- **MONTICELLI**. *Open Access Maced. J. Med. Sci.*, 2019, vol. 7 (7), 1077-1081
- **MONTICELLI et al.** *Open Access Maced. J. Med. Sci.*, 2019, vol. 7 (7), 1077-1081
- **SUNDARAM et al.** *Plast. Reconstr. Surg.*, 2015, vol. 136, 678-686
- **MONTICELLI et al.** *Open Access Macedonian J. Med. Sci.*, 2019, vol. 7 (7), 1077-1081